# EUROPEAN PATENT SPECIFICATION

(11) **EP 2 095 801 B1**
(45) Date of publication and mention of the grant of the patent: **12.10.2011**
(21) Application number: 09250601.3
(22) Date of filing: 02.03.2009
(51) Int. Cl.: A61F 13/26, B29C 57/10

(54) **Method and apparatus to form a spherical end of an elongated cylindrical tube, in particular of a tampon applicator**
Verfahren und Vorrichtung zur Bildung eines kugelförmigen Endes an einem länglichen zylindrischen Rohr, insbesondere an einem Tamponapplikator
Procédé et appareil pour former une extrémité sphérique d'un tube cylindrique allongé, en particulier d'un applicateur de tampon

(30) Priority: 29.02.2008 US 40016
(43) Date of publication of application: 02.09.2009
(73) Proprietor: Mcneil-PPC, Inc, Skillman, NJ 08558 (US)
(72) Inventor: Pelley, Kenneth A., Hopewell, New Jersey 08525 (US)
(74) Representative: Kirsch, Susan Edith

(56) References cited:
- EP-A- 0 260 932
- WO-A-03/013408
- GB-A- 1 484 912
- JP-A- 55 166 149
- US-A- 4 302 174
- US-A- 5 958 321
- US-A1- 2004 225 269

## Description

### FIELD OF THE INVENTION

This invention relates generally to a method and apparatus for forming elongated cylindrical tubes useful for inserting substances into a mammalian body and more particularly relates to a method and apparatus of forming a portion of a tampon applicator.

### BACKGROUND OF THE INVENTION

Insertion devices including tampon and nasal applicators, are generally known to be elongated cylindrical tubular structures that are used to house objects intended to be inserted in a body cavity and to expel the objects into the intended orifice. Applicators generally comprise an insertion member and a plunger. The object to be expelled from the applicator, such as a tampon, is positioned within the insertion member. The insertion member has a first end for insertion of the tampon and a second end for receipt of the plunger. Typically, the plunger is slightly smaller in diameter and is slidably positioned behind the tampon carried in the tampon applicator. To use the applicator, the user will position the first end appropriately, grasp the insertion member, and push the plunger into the insertion member towards the first end to insert the tampon. A variety of applicators have employed visual marks to determine when the contents of the applicator have been fully expelled.

Tampon applicators may be made from various materials including paperboard or plastic. Each type of material may have challenges during manufacture and use. For example, a tampon insertion member will generally incorporate surface features at the rear or gripper end to allow the user to hold the applicator more or less securely while ejecting the tampon from the opposite end of the applicator. Another way to aid insertion of the applicator into the body and also protect the contained tampon is to "dome" the insertion end of the applicator. A domed end provides for a smooth insertion and may help prevent contamination of the tampon prior to use.

GB 1484912 discusses a tampon inserter comprises a barrel portion with insertion end, cylindrical finger grip and plunger, the insertion end including a multiplicity of mangular segments which converge towards each other to form a dome and have a thickness less than that of the barrel portion so that they are flexible enough to allow the passage of tampon therethrough. The reduction in thickness between the barrel portion and the segments may be achieved either by injection moulding of thermoplastics or thermosetting plastic, or by use of laminated cardboard omitting some laminate layers at the insertion end.

In the past, doming or the rounding of the insertion end of the applicator has been performed in either 1) a two step heating and cooling process involving two separate dies or 2) a single step, requiring additional time for the single heated die to cool down. The two-step process requires a transfer of the applicator between hot and cold tools. The two-step process has drawbacks, which may include 1) contamination of the tools, especially if the heated tool retains degraded material such as plastic and 2) alignment issues, which may occur when the applicator is moved from one tool to the next tool. A further concern with such two-step processes is waste generated during process interruptions. Often the products trapped between the two steps are unsuitable for further processing upon restart of the system. An example of a two-step process is discussed in EP 0 260 932.

For these reasons, there remains a need for an efficient, single step process that quickly and cleanly domes the insertion end of an applicator. This would result in a better product, quicker production, and cost efficiency.

### SUMMARY OF THE INVENTION

I have invented an efficient, single step process that quickly and cleanly domes the insertion end of a heat-deformable applicator.

The invention provides a method for shaping a plurality of tampon applicators comprising a heat-deformable material includes the steps of heating a forming element forming surface; applying a portion of a first tampon applicator to the forming surface; shaping the portion of the first tampon applicator; cooling the forming surface; and removing the portion of the first tampon applicator from the forming surface. The forming element has a high thermal conductivity and a low thermal mass. The step of heating the forming element forming surface includes heating it to a temperature greater than the softening point of the heat-deformable material of the applicator portion. The step of cooling the forming element forming surface includes cooling it to a temperature less than the softening point of the heat-deformable material. The time required to heat and form the portion of the tampon applicator is less than about 90 seconds. These steps are repeated for subsequent tampon applicators.

The invention further provides an apparatus for shaping a tampon applicator comprising a mold body and means to provide at least a portion of the tampon applicator to the mold. The tampon applicator is at least partially formed of a heat-deformable material. The mold body has a forming element, a forming element fluid cavity, means to apply heat to the forming element, and means to provide cooling fluid through the forming element fluid cavity. The forming element has a high thermal conductivity and a low thermal mass and a forming surface. The forming element fluid cavity is adjacent to and in thermal contact with the forming element.

### BRIEF DESCRIPTION OF THE DRAWINGS

Fig. 1 is a side elevation of an applicator formed according to the present invention.
Fig. 2 is the side elevation of the applicator of Fig. 1, showing a tampon contained therein (in phantom lines).
Fig. 3 illustrates a prior art two-step process for forming a first end of an applicator into a domed shape.
Fig. 4 is a cross-section of a mold body of the apparatus of the present invention.
Fig. 5 is a cross-section of another mold body of the apparatus of the present invention.
Fig. 6 is a schematic diagram of a system according to the present invention.
Figs. 7A-B are cross-sections of a mold body of an apparatus of the present invention during heating (Fig. 7A) and cooling (Fig. 7B) operations.
Fig. 8 is a graph of the temperature profile resulting from the operation of a method according to the present invention as described in Example.

### DETAILED DESCRIPTION

As used herein, the term "tampon," refers to any type of absorbent structure that is inserted into the vaginal canal or other body cavities for the absorption of fluid therefrom, to aid in wound healing, or for the delivery of active materials, such as medicaments, or moisture. The tampon may be compressed into a generally cylindrical configuration in the radial direction, axially along the longitudinal axis or in both the radial and axial directions. While the tampon may be compressed into a substantially cylindrical configuration, other shapes are possible. These may include shapes having a cross section that may be described as rectangular, triangular, trapezoidal, semi-circular, hourglass, serpentine, or other suitable shapes. Tampons have an insertion end, withdrawal end, a length, a width, a longitudinal axis, a radial axis, and an outer surface. The tampon's length can be measured from the insertion end to the withdrawal end along the longitudinal axis. A typical compressed tampon for human use is 35-60 mm in length. A tampon may be straight or non-linear in shape, such as curved along the longitudinal axis. A typical compressed tampon is about 5 to about 20 mm wide. The width of a tampon, unless otherwise stated in the specification, corresponds to the distance across the largest cross-section perpendicular to the length of the tampon. The tampons of the present invention also include nasal tampons.

In addition to delivering menstrual tampons into the vaginal cavity, it should be noted that the tampon applicator of the present invention could be used to deliver any other type of absorbent or nonabsorbent object to any suitable cavity. For example, the tampon applicator of the present invention could be used to insert a treatment ovule or an incontinence device. An "incontinence device," as used herein refers to devices specifically designed, configured, and/or adapted for placement into a vagina in order to reduce the occurrence and/or severity of female urinary incontinence. While incontinence devices are typically made of non-absorbent materials, at least partially absorbent materials may also be used. However, because there is no intent to absorb bodily fluids, and because the incontinence devices are adapted and configured to provide structural support to the musculature and body tissues, incontinence devices are readily distinguishable from tampons. Non-limiting specific examples of such include any known hygienically designed applicators that are capable of receiving a tampon may be used for insertion of a tampon, including the so-called telescoping, tube and plunger, and the compact applicators, an applicator for providing medicament to an area for prophylaxis or treatment of disease, a spectroscope containing a microcamera in the tip connected via fiber optics, a speculum of any design, a tongue depressor, a tube for examining the ear canal, a nano-hollow pipe for guiding surgical instruments, and the like.

The term "expelled," as used herein is meant the tampon is forced out of the insertion portion of the tampon applicator.

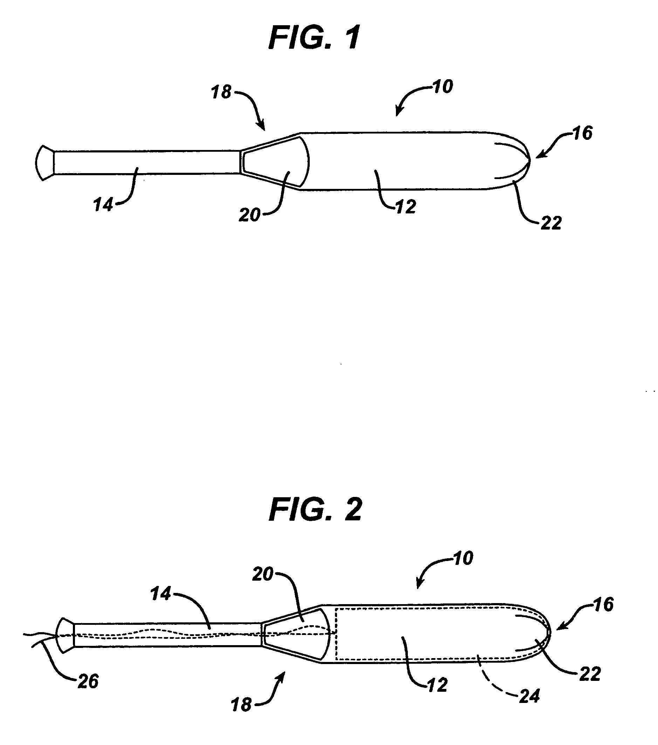

The Figs. 1 and 2 show embodiments of the tampon applicator of the present invention. The present invention, however, is not limited to a structure having the particular configurations shown in the drawings or discussed herein. The tampon applicator of the present invention can have any configuration or size as long as the ease of delivery of the tampon into the body is retained.

The tampon applicator 10 has a pre-expelled state as shown in Figs. 1 and 2. As shown, applicator 10 has an insertion member 12 and a plunger 14. Insertion member 12 is hollow and has a first or insertion end 16 dimensioned for insertion into the body cavity (specifically the vaginal cavity of a female user), a second end 18 positioned oppositely to the first end 16 and a gripping portion 20. The first end 16 further has a plurality of petals 22.

The insertion member 12 is sized and configured to house an insertable element, such as an absorbent tampon 24 having a withdrawal string 26. As stated above, the insertion member 12 preferably has a substantially smooth exterior surface or exterior surface that exerts low drag with vaginal body tissue that will facilitate insertion of the insertion member 12 into a woman's vagina. When the exterior surface is smooth and/or slippery, the insertion member 12 will easily slide into a woman's vagina without subjecting the internal tissues of the vagina to abrasion. The insertion member 12 can be coated to give it a high slip characteristic. Wax, polyethylene, a combination of wax and polyethylene, cellophane, clay, mica and other lubricants are representative coatings that can be applied to the insertion member 12 to facilitate comfortable insertion.

The applicator 10 of the present invention can have geometries or cross-sections that are useful to contain the object to be inserted. Often, the shape of the tampon 24 contained suggests the shape of the insertion member 12, but departures from this general rule can be made such that a cylindrical tampon 24 can be housed in a rectangular shaped applicator, for example. The insertion member 12 and plunger 14 can take on numerous cross-sectional shapes including without limitations, circular, oval, polygonal (e.g. trapezoidal, rectangular, triangular) and the like. In addition the insertion member 12 and plunger 14 can be substantially elongated, such as in a linear fashion, curved or flexible, or it can take on other shapes that are apparent to one of ordinary skill in the art. Further, the insertion member 12 and plunger 14 can be substantially elongated, curved or flexible, or it can take on other shapes that are apparent to one of ordinary skill in the art. Some examples of applicator shapes are described in WO 2004/024193 published by Lecan, et al. on Mar. 25, 2004, and European Patent Application No, 1101473 published by Mitsuhiro, et al on May 23, 2001.

The insertion end 16 of the insertion member 12 can be substantially closed and can comprise petals, corrugations, or pleats. During insertion, when the tampon 24 is pushed upward by the plunger 14, the petals 22 open and to let the tampon 24 through, into the vagina.

The applicator devices of the present invention include heat-deformable materials and may also include other materials generally known to those of ordinary skill in the art. In one embodiment, the insertion member 12 and plunger 14 can be formed of plastic, e.g., by injection molding, blow-molding, extruding, or otherwise formed from flexible plastic, such as thermoformed from plastic sheet or folded or wound from plastic film. Additionally, the applicator device may be made from biodegradable plastics such as those disclosed in Dabi et al., U. S. Pat. No. 5,910,520. If desired, the sleeves may also include a paper or cardboard laminate in a manner known to those skilled in the art.

The different tampon applicator parts can be constructed from different materials and processes. The tampon applicator 10 or any part of the tampon applicator can be formed of a spirally wound, convolutedly wound, or longitudinally seamed hollow tube that is formed from paper, paperboard, cardboard, or any combinations thereof. A coating may be provided to the surface.

The tampon applicator 10 or any part of the tampon applicator can be constructed from a single ply of material or be formed from two or more plies that are bonded together to form a laminate. However, at least one ply of the laminate must comprise (including via coating) a heat-deformable material. The use of two or more plies or layers is preferred for it enables the manufacturer to use certain materials in the various layers that can enhance the performance of the tampon applicator or any part of the tampon applicator. When two or more plies are utilized, all the plies can be spirally wound, convolutedly wound, or longitudinally seamed to form an elongated cylinder. The tampon applicator or any part of the tampon applicator can be constructed using a smooth thin ply of material on the outside or exterior surface that surrounds a coarser and possibly thicker ply.

The plies forming the tampon applicator or any part of the tampon applicator can be held together by an adhesive, such as glue, heat, pressure, ultrasonic, or any combinations thereof. The adhesive can be either water-soluble or water-insoluble. A water-soluble adhesive is preferred for environmental reasons in that the tampon applicator or any part of the tampon applicator will quickly break apart when it is immersed in water. Such immersion will occur should the tampon applicator or any part of the tampon applicator be disposed of by flushing it down a toilet. Exposure of the tampon applicator or any part of the tampon applicator to a municipal's waste treatment plant wherein soaking in water, interaction with chemicals, and agitation all occur, will cause the tampon applicator or any part of the tampon applicator to break apart and evenly disperse in a relatively short period of time.

Typical dimensions for the tubular elements useful in tampon applicators include a length of about 5 to 8 cm, a diameter of about 8 to about 20 mm, and thicknesses of about 0.1 to 0.6 mm. Preferably, the diameter of the plunger 14 is less than the diameter of the insertion member 12 to allow for a telescopic arrangement as shown in Figs. 1 and 2.

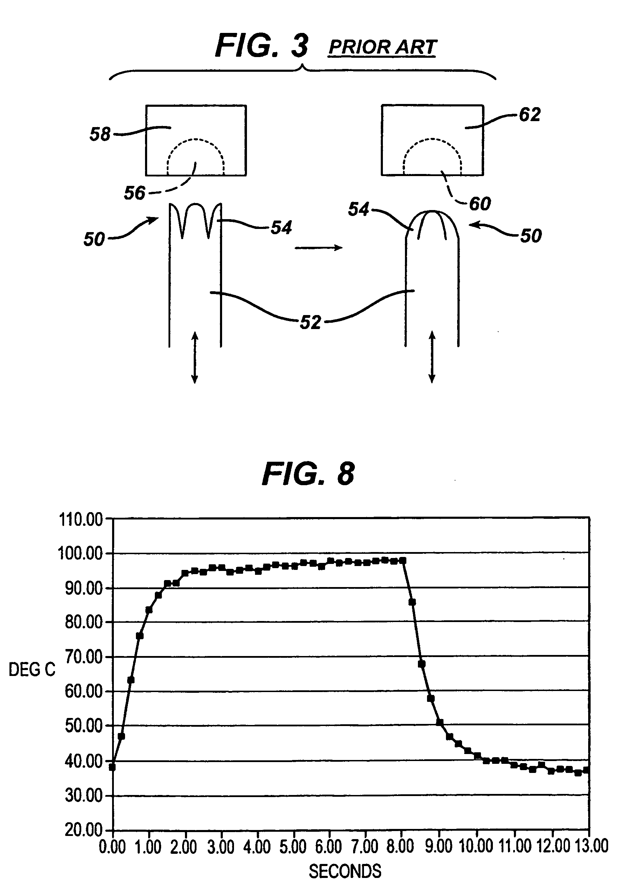

Fig. 3 shows the basics of a conventional two-step process (prior art). The insertion member and the plunger can be supplied from different sources but may be assembled prior to doming. A tampon may be contained within the assembly. The first end 50 of insertion member 52 is open and provided with petals 54. The open first end 50 of the insertion member 52 is aligned and placed within the heating chamber 56 of a mold 58. While in the chamber 56, the first end 50 is subjected to a temperature greater than the softening point of the heat-deformable material and to shaping pressure. The petals 54 form, in the figures shown, a "dome", which is substantially a hemispherical shape. While a preferred shape is hemispherical, other shapes may be formed such as a bullet tip. The domed first end 50 is then removed from the heating chamber 56 and indexed to a cooling chamber 60. The domed first end 50 is now placed within cooling chamber 60 of the cooling apparatus 62, which removes excess heat from the domed first end 50, and the heat-deformable material takes a more permanent form. This process requires two steps. Drawbacks to the two-step process include hot tool contamination by melted or charred insertion member material and hot (and thereby soft) petals sticking to the hot tool upon removal of the domed petal end resulting in a malformed end. Another issue with the two-step process is the potential for misalignment of the insertion member when it goes from the heating chamber and into the cooling chamber. A further concern with such two-step processes is waste generated during process interruptions. Often the products trapped between the two steps are unsuitable for further processing upon restart of the system.

The present, one-step invention provides advantages over the prior art two-step process. The present invention provides a one-step process in a mold that heats, forms, and sets the domed first end of the insertion member. This also permits the doming process to be completed during process interruptions, and no incomplete doming workpieces are trapped between process steps. Thus, this one-step process provides significant waste reduction.

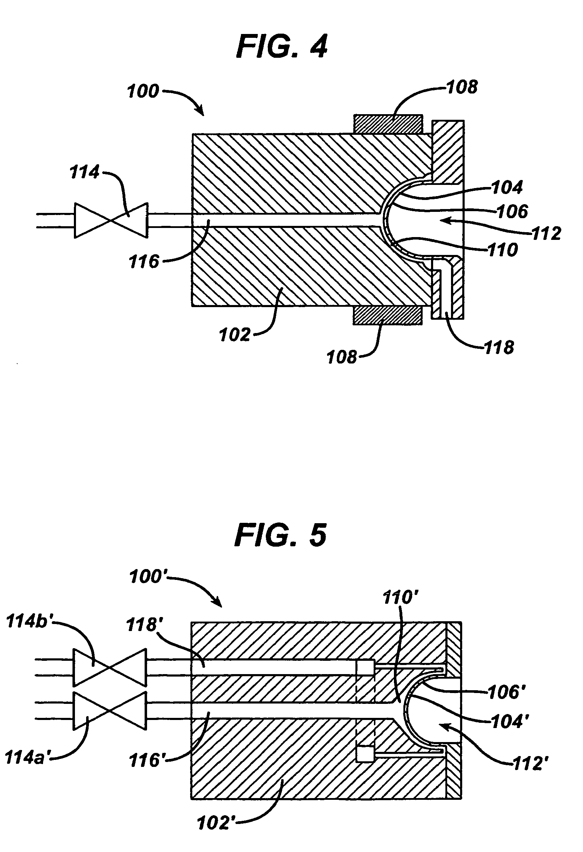

The one-step process shown in Figs. 4-7 employs a thin-walled mold 100, e.g., a mold having a body 102 that houses a high thermal conductivity forming element 104 to transfer thermal energy to form and set a desired dome shape to the insertion end of a tampon applicator. Initially, the forming surface 106 is heated to a target temperature, greater than the softening point of a heat-deformable material of the tampon applicator, a portion of the tampon applicator is inserted into the heated thin-walled mold 100 and held stationary to soften petals and form them into a dome shape. The forming element 104 is cooled to remove heat from the domed portion of the tampon applicator, thereby setting the domed form of the insertion end. After the domed end is cooled, it is removed from the mold 100.

Fig. 4 shows a cross-section of a mold body. The mold body 102 is heated with an electric band heater 108 to the target temperature. Heat is transferred across a thin cavity (small air gap of the fluid cavity 110) to the forming element 104. When the forming element 104 has reached the target temperature, an insertion end of the applicator (not shown) is inserted into the mold cavity 112, contacting forming surface 106 and allowed to come to softening temperature. Once the petals of the insertion end have softened and been formed by the forming surface 106, a valve 114 is opened and a cooling fluid, (e.g., cold air) is supplied through a cooling supply fluid conduit 116 to the fluid cavity 110 of the mold. The cooling fluid passes through the body 102 of the mold to remove heat from the forming element 104 as the fluid passes through the fluid cavity 110, and it exits the body 102 of the mold at port 118. The forming surface is preferably heated to a temperature of 100 ± 5° C. Cooling fluid is provided for preferably about one minute or until the mold reaches the preferred temperature of 65 ± 5° C. The electric band heater 108 may be switched off during the cooling cycle, alternatively, it may remain on continuously.

The time required complete a heating and cooling cycle can preferably take approximately two minutes. By optimizing the choice of materials used to make the mold portions, one may influence the cycle time. Additionally, the choice of cooling fluid may affect the cycle time.

The forming body may preferably be heated for 1 minute and cooled for 1 minute, resulting in a cycle time of 2 minutes.

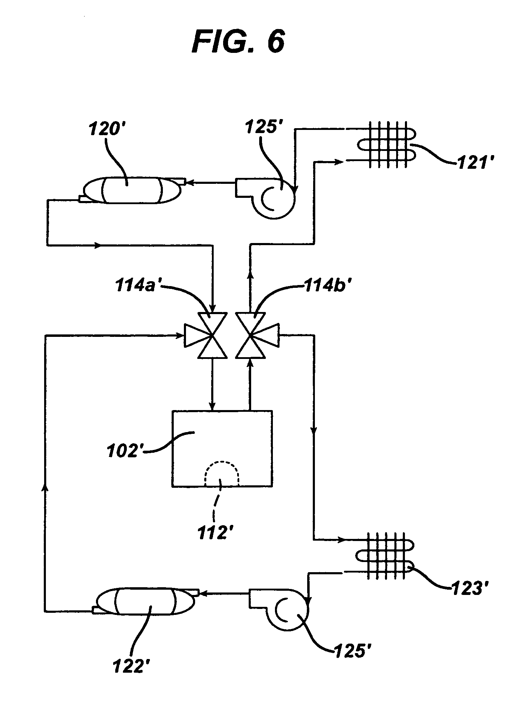

Another mold body is shown in Fig. 5 (and schematically in Fig. 6). Heating and cooling of the forming element 104' is accomplished by directing heating and cooling fluids through a single fluid intake conduit 116' to the fluid cavity 110'. As needed, fluid is circulated from either a heating fluid reservoir 120' (heated by heater 121') or cooling fluid reservoir 122' (cooled by chiller 123') through the operation of pumps 125'. The forming element 104' is heated to a target temperature and heat is transferred to the forming surface 106'. When the forming element 104' has reached the target temperature, the process described above is repeated with the heating and cooling of the forming element 104' controlled by supply of heating or cooling fluid by operation of the supply valve 114a' and return valve 114b'. The fluid exits the mold body 102' through exit port 118'.

The use of heating fluids reduces the time required to heat the forming element compared with an external electric band heater. Thus the complete cycle time is also reduced, and a complete heating and cooling cycle can take less than 30 seconds, and even less than 20 seconds. Again, optimizing the choice of materials used to make the mold portions may influence the cycle time. We have noticed that the heating step often takes more time than the cooling step.

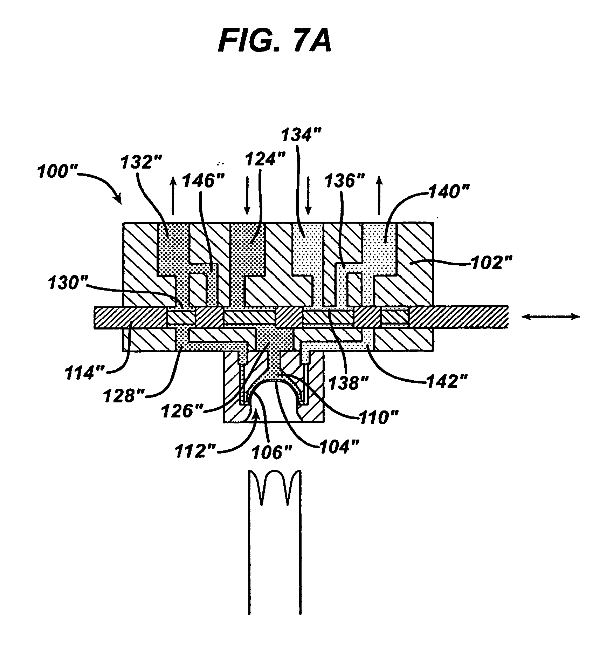

Figs. 7A-B show the operation of a mold body in which a fluid valve is incorporated within the mold body 102". This internal valve (e.g., a spool valve 114") replaces external supply valves and allows for a steady, on-demand supply of appropriate heating or cooling fluid. Additionally, the common fluid path is shortened, which minimizes the mixing of heating and cooling fluids. This, in turn, can reduce the cycle time.

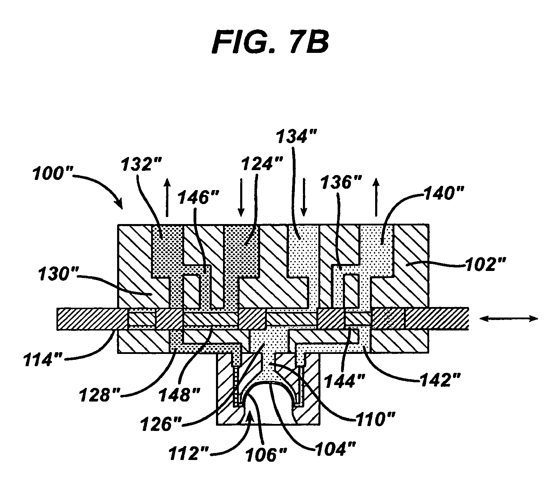

As needed, fluid is moved from either a heating fluid reservoir or cooling fluid reservoir (as selected by operation of valve 114"). As shown in Fig. 7A, valve 114" is positioned to provide circulation of heating fluid (darker shading) through heating fluid intake 124", common fluid supply conduit 126" to fluid cavity 110". The heating fluid exits the fluid cavity 110" through heating fluid exit conduit 128" and heating fluid exit region 130" of valve 114" and to heating fluid exhaust port 132". During the heating phase of operation, cooling fluid (lighter shading) circulates through cooling fluid intake 134", cooling fluid bypass conduit 136", cooling fluid bypass region 138" of valve 114" and to cooling fluid exhaust port 140". During the cooling phase (shown in Fig. 7B), 114" is positioned to provide circulation of cooling fluid through cooling fluid intake 134", common fluid supply conduit 126" to fluid cavity 110". The cooling fluid exits the fluid cavity 110" through cooling fluid exit conduit 142" and cooling fluid exit region 144" of valve 114" and to cooling fluid exhaust port 140". During the cooling phase of operation, heating fluid circulates through heating fluid intake 124", heating fluid bypass conduit 146", heating fluid bypass region 148" of valve 114" and to heating fluid exhaust port 132".

As described in respect of the mold body illustrated in Figure 4, the forming element 104" is heated to a target temperature and heat is transferred to the forming surface 106". When the forming element 104" has reached the target temperature, the process described above is repeated with the heating and cooling of the forming element 104" controlled by operation of internal valve 114", as described above.

The use of heating fluids and the internal valve further reduces the time required to effectively switch between heating and cooling phases of the process compared with the system described in Figure 5. Thus the complete cycle time is also reduced, and a complete heating and cooling cycle can take less than 20 seconds, and even less than 15 seconds. Again, optimizing the choice of materials used to make the mold portions may influence the cycle time.

### Example 1

Using a prototype of a mold body (as shown in Fig. 7A) formed of low density polyethylene, a trial run was set up using the following conditions:
- Light hydraulic fluid was used for the heating and cooling fluid.
- The target temperature for the heating fluid was 100° C, resulting in a forming element surface temperature of nearly 100° C. The target temperature for the cooling fluid was room temperature, approximately 25-30 °C, resulting in a forming element surface temperature of less than 40° C.

The resulting temperature profile is shown in Fig. 8. The results show that the mold achieved both the target heating and cooling temperatures within 3 seconds of switching the spooling valve, and the total cycle time is about 13 seconds.

## Claims

1. A method for shaping a plurality of tampon applicators (10) comprising a heat-deformable material, the method comprising the steps of:
a) heating a forming element (104) forming surface (106) to a temperature greater than a heat-deformable material softening point, wherein the forming element has a high thermal conductivity and a low thermal mass;
b) applying a portion of a first tampon applicator to the forming surface;
c) shaping the portion of the first tampon applicator;
d) cooling the forming surface to a temperature less than the heat-deformable material softening point; and
e) removing the portion of the first tampon applicator from the forming surface;
f) repeating steps a) through e) for a second tampon applicator;
wherein the duration of applying the portion of each tampon applicator to the forming surface having a temperature greater than the heat-deformable material softening point is less than 90 seconds.

2. The method of Claim 1, wherein the portion of each tampon applicator applied to the forming surface comprises a first end of the tampon applicator, and the first end of the tampon applicator comprises a plurality of petals (22) that are shaped into a domed first end of the tampon applicator.

3. The method of Claim 1 or Claim 2, wherein the step of heating the forming element forming surface comprises directing a heating fluid to a forming element fluid cavity (110) adjacent to and in thermal contact with the forming element.

4. The method of any preceding Claim, wherein the step of cooling the forming element forming surface comprises directing a cooling fluid to a forming element fluid cavity (110) adjacent to and in thermal contact with the forming element.

5. The method of any preceding Claim, wherein steps a)-e) are completed within 2 minutes

6. The method of any preceding Claim, wherein the first tampon applicator comprises a polyethylene material and steps a)-e) are completed within 20 seconds.

7. Apparatus for shaping a tampon applicator (10) comprising a heat-deformable material, the apparatus comprising:
a) a mold body (102) having:
i) a forming element (104) having a high thermal conductivity and a low thermal mass and a forming surface (106);
ii) a forming element fluid cavity (110) adjacent to and in thermal contact with the forming element;
iii) means to apply heat to the forming element; and
iv) means to provide cooling fluid through the forming element fluid cavity (110); and
b) means to provide at least a portion of the tampon applicator to the mold.

8. The apparatus of Claim 7, wherein the means to apply heat to the forming element comprises a heat supply fluid conduit (116) operatively connectable to the forming element fluid cavity.

9. The apparatus of Claim 7 or Claim 8, wherein the means to apply provide cooling fluid to the forming element fluid conduit comprises a cooling supply fluid conduit (116) operatively connectable to the forming element fluid cavity.

10. The apparatus of Claim 7, Claim 8 or Claim 9, wherein the forming element comprises a thin-wall forming surface.

## Patentansprüche

1. Verfahren zur Formung einer Vielzahl von Tamponapplikatoren (10), das ein wärmeverformbares Material umfasst, wobei das Verfahren die folgenden Schritte umfasst:
a) Erwärmen der Formgebungsfläche (106) eines Formgebungselementes (104) auf eine Temperatur, die größer ist als ein Erweichungspunkt eines wärmeverformbaren Materials, wobei das Formgebungselement eine hohe Wärmeleitfähigkeit und eine geringe thermische Masse aufweist;
b) Aufbringen eines Abschnitts eines ersten Tamponapplikators auf die Formgebungsfläche;
c) Formen des Abschnitts des ersten Tamponapplikators;
d) Kühlen der Formgebungsfläche auf eine Temperatur, die niedriger als der Erweichungspunkt des wärmeverformbaren Materials ist; und
e) Entfernen des Abschnitts des ersten Tamponapplikators von der Formgebungsfläche;
f) Wiederholen der Schritte a) bis einschließlich e) für einen zweiten Tamponapplikator;
wobei die Dauer des Aufbringens des Abschnitts von jedem Tamponapplikator auf die Formgebungsfläche, die eine Temperatur aufweist, die größer als der Erweichungspunkt des wärmeverformbaren Materials ist, weniger als 90 Sekunden beträgt.

2. Verfahren nach Anspruch 1, wobei der von jedem Tamponapplikator auf die Formgebungsfläche aufgebrachte Abschnitt ein erstes Ende des Tamponapplikators aufweist, und wobei das erste Ende des Tamponapplikators eine Vielzahl von Blütenblättern (22) aufweist, die in ein haubenförmiges erstes Ende des Tamponapplikators hineingeformt sind.

3. Verfahren nach Anspruch 1 oder Anspruch 2, wobei der Schritt der Erwärmung der Formgebungsfläche des Formgebungselementes das Führen einer Heizflüssigkeit zu einem Flüssigkeitshohlraum (110) des Formgebungselementes umfasst, der an das Formgebungselement angrenzt und damit in thermischem Kontakt steht.

4. Verfahren nach einem der vorhergehenden Ansprüche, wobei der Schritt des Kühlens der Formgebungsfläche des Formgebungselementes das Führen einer Kühlflüssigkeit zu einem Flüssigkeitshohlraum (110) des Formgebungselementes umfasst, der an das Formgebungselement angrenzt und damit in thermischem Kontakt steht.

5. Verfahren nach einem der vorangehenden Ansprüche, wobei die Schritte a)-e) innerhalb von 2 Minuten abgeschlossen werden.

6. Verfahren nach einem der vorangehenden Ansprüche, wobei der erste Tamponapplikator ein Polyethylenmaterial aufweist, und die Schritte a)-e) innerhalb von 20 Sekunden abgeschlossen werden.

7. Vorrichtung zur Formung eines Tamponapplikators (10), die ein wärmeverformbares Material aufweist, wobei die Vorrichtung Folgendes aufweist:
a) Einen Gießformkörper (102), der Folgendes aufweist:
(i) Ein Formgebungselement (104) mit einer hohen Wärmeleitfähigkeit und einer geringen thermischen Masse, und mit einer Formgebungsfläche (106);
(ii) einen Flüssigkeitshohlraum (110) des Formgebungselementes, der an das Formgebungselement angrenzt und damit in thermischem Kontakt steht;
(iii) eine Einrichtung zum Aufbringen von Wärme auf das Formgebungselement; und
(iv) eine Einrichtung zur Bereitstellung von Kühlflüssigkeit durch den Flüssigkeitshohlraum (110) des Formgebungselementes; und
b) eine Einrichtung zur Bereitstellung mindestens eines Abschnitts des Tamponapplikators für die Gießform.

8. Vorrichtung nach Anspruch 7, wobei die Einrichtung zum Aufbringen von Wärme auf das Formgebungselement eine Wärmeflüssigkeitszufuhrleitung (116) aufweist, die mit dem Flüssigkeitshohlraum des Formgebungselementes in Wirkverbindung bringbar ist.

9. Vorrichtung nach Anspruch 7 oder Anspruch 8, wobei die Einrichtung zur Bereitstellung von Kühlflüssigkeit für das Formgebungselement eine Kühlflüssigkeitszufuhrleitung (116) aufweist, die mit dem Flüssigkeitshohlraum des Formgebungselementes in Wirkverbindung bringbar ist.

10. Vorrichtung nach Anspruch 7, Anspruch 8 oder Anspruch 9, wobei das Formgebungselement eine dünnwandige Formgebungsfläche aufweist.

## Revendications

1. Procédé pour former une pluralité d'applicateurs de tampon (10) comprenant une matière déformable à la chaleur, le procédé comprenant les étapes suivantes:
a) chauffer une surface de formage (106) d'un élément de formage (104) à une température qui est supérieure au point de ramollissement de la matière déformable à la chaleur, dans lequel l'élément de formage présente une conductivité thermique élevée et une masse thermique réduite;
b) appliquer une partie d'un premier applicateur de tampon sur la surface de formage;
c) former la partie du premier applicateur de tampon;
d) refroidir la surface de formage à une température qui est inférieure au point de ramollissement de la matière déformable à la chaleur; et
e) enlever la partie du premier applicateur de tampon de la surface de formage; et
f) répéter les étapes a) à e) pour un deuxième applicateur de tampon,
dans lequel la durée d'application de la partie de chaque applicateur de tampon sur la surface de formage qui présente une température supérieure au point de ramollissement de la matière déformable à la chaleur est inférieure à 90 secondes.

2. Procédé selon la revendication 1, dans lequel la partie de chaque applicateur de tampon qui est appliquée à la surface de formage comprend une première extrémité de l'applicateur de tampon, et la première extrémité de l'applicateur de tampon comprend une pluralité de pétales (22) qui sont configurés en une première extrémité bombée de l'applicateur de tampon.

3. Procédé selon la revendication 1 ou la revendication 2, dans lequel l'étape de chauffage de la surface de formage de l'élément de formage comprend la direction d'un fluide de chauffage vers une cavité de fluide d'élément de formage (110) à proximité de et en contact thermique avec l'élément de formage.

4. Procédé selon l'une quelconque des revendications précédentes, dans lequel l'étape de refroidissement de la surface de formage de l'élément de formage comprend la direction d'un fluide de refroidissement vers une cavité de fluide d'élément de formage (110) à proximité de et en contact thermique avec l'élément de formage.

5. Procédé selon l'une quelconque des revendications précédentes, dans lequel les étapes a) à e) sont accomplies en 2 minutes maximum.

6. Procédé selon l'une quelconque des revendications précédentes, dans lequel le premier applicateur de tampon comprend une matière de polyéthylène, et les étapes a) à e) sont accomplies en 20 secondes maximum.

7. Appareil pour former un applicateur de tampon (10), comprenant une matière déformable à la chaleur, l'appareil comprenant:
a) un corps de moule (102), comprenant:
i) un élément de formage (104) qui présente une conductivité thermique élevée et une masse thermique réduite, et une surface de formage (106);
ii) une cavité de fluide d'élément de formage (110) à proximité de et en contact thermique avec l'élément de formage;
iii) des moyens pour appliquer de la chaleur à l'élément de formage; et
iv) des moyens pour faire passer un fluide de refroidissement à travers la cavité de fluide d'élément de formage (110); et
b) des moyens pour introduire au moins une partie de l'applicateur de tampon dans le moule.

8. Appareil selon la revendication 7, dans lequel les moyens pour appliquer de la chaleur à l'élément de formage comprennent un conduit de fluide de fourniture de chaleur (116) qui peut être connecté de façon opérationnelle à la cavité de fluide d'élément de formage.

9. Appareil selon la revendication 7 ou la revendication 8, dans lequel les moyens pour fournir un fluide de refroidissement au conduit de fluide d'élément de formage comprennent un conduit de fluide de fourniture de refroidissement (116) qui peut être connecté de façon opérationnelle à la cavité de fluide d'élément de formage.

10. Appareil selon la revendication 7, la revendication 8 ou la revendication 9, dans lequel l'élément de formage comprend une surface de formage à paroi mince.
